# EUROPEAN PATENT SPECIFICATION

(11) **EP 1 244 493 B1**
(45) Date of publication and mention of the grant of the patent: **15.06.2005**
(21) Application number: 00985622.0
(22) Date of filing: 20.12.2000
(51) Int. Cl.: A61M 29/00, A61M 25/00, A61B 17/12, A61F 2/06

(54) **DEVICE FOR CONTROLLING EXTRA-VASCULAR HAEMORRHAGE**
GERÄT ZUR KONTROLLE DER EXTRA-VASCULAR-BLUTUNGEN
DISPOSITIF PERMETTANT DE CONTENIR UNE HEMORRAGIE EXTRAVASCULAIRE

(30) Priority: 23.12.1999 GB 9930654
(43) Date of publication of application: 02.10.2002
(73) Proprietor: Halpin, Richard Michael Bestall, Melrose TD6 0HB (GB)
(72) Inventor: Halpin, Richard Michael Bestall, Melrose TD6 0HB (GB)
(74) Representative: Newell, Campbell
(86) International application number: PCT/GB2000/004877
(87) International publication number: WO 2001/047594

(56) References cited:
- WO-A-95/05860
- WO-A-97/40889
- WO-A-99/20324
- GB-A- 1 538 737
- US-A- 4 832 688
- US-A- 5 397 307
- US-A- 5 843 116
- US-A- 5 954 741

## Description

The present invention relates to devices for controlling extra-vascular haemorrhage and, in particular, to an inflatable device for use as an intravascular haemostatic device.

Damage of injury to blood vessels may lead to extravasation (bleeding out from blood vessels). Such internal bleeding leads, in turn, to loss of blood-pressure in the patient having the damaged or injured blood vessel. Extravasation is particularly dangerous to the patient where the damaged vessels are the retro-hepatic vena cava and/or the hepatic veins as these blood vessels are very difficult to reach by a surgeon for repair thereof. These blood vessels are located close to the spine and behind the liver, and for a surgeon to repair a damaged vessel of this type it is first necessary for the surgeon to be able to have relatively unhindered access to the vessel. Usually, access to damaged blood vessels may be achieved by relatively straightforward dissection. However, in the case of damage to the retro-hepatic vena cava or the hepatic veins the emergency dissection required is particularly difficult as the surgeon must generally dissect anteriorly with the bulk of the liver directly in the way with the result that access is obstructed. This is a very time consuming procedure and furthermore is not a procedure encountered on a regular basis by the majority of surgeons due to the relatively infrequent occurrence of damage to these vessels (retro-hepatic vena cava and hepatic veins).

A further complication in these types of procedures is that further blood loss may result from the surgery procedure itself (iatrogenic damage). This complication may also be further aggravated, in part, by the surgeon performing such an unfamiliar procedure.

Procedures to repair damaged blood vessels must in general be performed as quickly as possible to minimise the degree of extravasation. Control of extravasation must be achieved at the earliest opportunity i.e. within a matter of a few minutes at most, especially where a major blood vessel is damaged. It is possible for total exsanguination to occur within two or three minutes. Injury or damage to the liver itself, particularly where there is severe disruption to the liver architecture by penetrating injury, avulsion of the liver, or maceration of the liver itself resulting in loss of integrity of the liver's own blood vessels, and thus haemorrhage, is associated with high mortality and morbidity. Difficulty in achieving timely control of extravasation results generally in blood flowing back along the hepatic veins and out through the damaged/injured liver and into the peritoneum. The difficulty in controlling extravasation is inter alia a direct result of the difficulty of the surgeon gaining quick access to the injury site and then establishing control of the extravasation. It is also difficult to both operate and control haemorrhage at the same time.

Once a surgeon has gained access to the injured or damaged vessel or organ, in order to repair the damaged portion thereof, it is necessary to at least temporarily control the extravasation from the injury or damaged portion of the vessel or organ. This at least temporary control of extravasation allows the surgeon to effect a repair to the injured/damaged vessel or organ. In techniques where such temporary control is imposed, the organs or other body part(s) supplied by blood from the damaged vessel (or for blood returning to the heart) may be starved of blood (ischaemia). This can have serious consequences such as organ damage, or mortality. Temporary control of extravasation from the damaged/injured vessel/organ must therefore not substantially prevent blood flow to or from an organ or body to be supplied therefrom, if these serious complications are to be avoided.

Previously, temporary control of extravasation has been attempted using shunting techniques. "Insertion of a Retrohepatic Vena Cava Balloon Shunt Through the Saphenofemoral Junction" (OJ McAnena, EE Moore & FA Moore), The American Journal of Surgery, Vol.158, November 1989 described a retrohepatic vena cava balloon shunt. The device is relatively big and clumsy, and relies on the use of an intercostal chest drain, normally used for draining air, blood, pus or other fluid out of the chest, and an inflatable balloon to occlude the damaged portion of the vessel. This device also requires time-consuming dissection of the patient and relatively complex surgical manoeuvres in order to insert and use the device. Only surgeons and theatre staff practised in the technique of insertion which is required would be able to use the device competently. Moreover, blood flowing in the damaged vessel must ingress into the shunting tube through small holes provided in a section of the tube close to the balloon, and exit from more small holes in the shunting tube after the balloon, in order to bypass the balloon. This results in restriction of blood flow in the vessel which is a major disadvantage. Particularly in shocked patients where blood circulating volume needs to be restored as soon as possible, any restriction of flow will have a deleterious effect and may be primarily responsible for the death of the patient.

It is an object of the present invention to avoid or minimise one or more of the abovementioned problems.

According to a first aspect of the invention, there is provided an inflatable device for inserting, in a substantially deflated state, into a blood or other biological fluid carrying vessel of a human or animal, the device having an inflated state in which it defines elongate open-ended tube means via which the blood or other biological fluid in the vessel flows through the device in use thereof.

More specifically, but not exclusively, the invention provides an inflatable device for inserting, in a substantially deflated state, into a damaged blood or other biological fluid carrying vessel of a human or animal, and having an inflated state for substantially occluding the damaged portion of the vessel so as to mmmise, preferably substantially prevent, leakage from said damaged portion, wherein the device in its inflated state defines elongate open-ended tube means via which the blood or other biological fluid in the vessel flows through the device in use thereof, whereby said damaged portion of the vessel is bypassed.

One advantage of the invention is that the device can be located within the blood/biological fluid conveying vessel at the site of the damaged portion thereof, and then inflated to its inflated condition so as to occlude the damaged portion of the blood/biological fluid conveying vessel, thereby preventing extravasation therefrom while at the same time allowing blood/biological fluid to flow relatively unrestricted through the device. In this manner normal, or near normal, anatomical and physiological blood flow in the vessel can be maintained. This is particularly important for shocked patients, where blood has extravasated or come out of the vessels and been lost to the circulation. Moreover, the tubular form of the device also facilitates the occlusion of cuts, tears or other apertures in the vessel of various sizes and shapes. The device of the present invention may also be used instead of techniques such as atriocaval shunting where access to the patient's heart is required for insertion of, for example, a chest tube/drain into the pericardial cavity.

Using conventional equipment, such shunting requires a large degree of dissection to provide access to the heart. The device of the present invention reduces the degree of dissection necessary for insertion of a chest tube compared to that required for inserting the device into the Inferior Vena Cava (IVC) below the hepatic veins and moving one end of the device inside the IVC and into the pericardium.

The elongate tube means defined by the inflated device is preferably substantially cylindrical in form, having an inlet end and an outlet end and passage means, comprising at least one passage, defined therebetween, through the longitudinal length of the device. The cylindrical form provides a close fit with the lumen of the damaged vessel. The diameter of said passage means is preferably sufficiently large that the blood (or other biological fluid) may flow relatively unhindered through the device. Thus, the diameter of said passage means may be at least 50%, preferably greater than 50%, most preferably in the range of 90 to 95%, of the outer diameter of the inflated device.

The device of the present invention operates in the manner of an inflatable sleeve which may be inserted intra-vascularly and located at an injury site e.g. a rupture in a blood vessel, from which blood is escaping. The size of the inflated device is chosen such that upon inflation of the device, an outer wall of the device presses against the intima of the blood vessel at the rupture, forming a seal which prevents blood from escaping from the rupture in the vessel while at the same time the tubular form of the inflated device allows blood to continue to flow relatively unhindered through the device, following its natural flow path in the vessel.

The inflatable device preferably comprises one or more substantially collapsible walls defining a substantially enclosed inflatable volume. For example, the device may conveniently comprise a balloon shaped so as to define said elongate tube means when inflated. The device can be inflated by injecting saline or another physiologically acceptable fluid, or combination of fluids, into the enclosed inflatable volume.

The device may be generally circular in cross-section. Alternatively, it may be formed with an elliptical or irregular cross-section, according to the particular shape or configuration of the damaged blood or biological fluid carrying vessel in which the device is to be used.

It will be generally appreciated that the relative length and diameter dimensions of the device may be varied (at manufacture) according to the particular requirements of the use to which the device may be put. For example, where the device is to be deployed within a relatively large internal diameter vessel such as the inferior vena cava (IVC) or aorta, it would be necessary to use a device with correspondingly large diameter; conversely where the device is to be used in a relatively small internal diameter vessel such as femoral or subclavian arteries, a correspondingly small diameter is required.

The longitudinal length of the inflatable device may be varied similarly (at manufacture) according to need. For example, where the area of injury is quite extensive along the length of the blood vessel, then it would generally be necessary to use a device of a length sufficient to extend to at least the same length as the injury area of the vessel (and preferably slightly greater length).

The device of the present invention is preferably provided with a working channel (or conduit) which may conveniently be substantially aligned with a longitudinal axis of the device. More preferably, the working channel or conduit is formed and arranged to extend at least part-way through the longitudinal extent of the device. Desirably, the working channel or conduit formed and arranged for receiving therein at least one of : fluids, devices and a guide-wire. It will of course be appreciated that where the working channel/conduit can be utilised to locate or deliver said fluids, devices or guide wire to a location within the device (such as the passage means) or to a location exterior to the device depending upon *inter alia* the longitudinal extent of the working channel/conduit. Where, for example, the working channel/conduit is intended to be used for receiving a said guide-wire or device, it will be appreciated that the working channel need not be a totally enclosed channel (such as a pipe), but may by a channel being of a C-shaped cross-section which would be sufficient to retain the guide-wire or device therein, but would not be suitable to receive a fluid therethrough. According to one embodiment of the invention, the working channel is the hollow interior of a central elongate conduit or similar such structure which may be integrally formed with the inflatable device, or may be fixed to the device at at least one point therealong, with opposite end portions of the elongate conduit protruding from respective opposite ends of the tube means defined by the inflated device. A first one of said protruding end portions is preferably relatively long, for example up to approximately one metre long, while the second end portion protrudes only a very short length beyond the tubular form of the inflated device (e.g. only a few centimetres). Said first protruding end portion is preferably provided with a first self-sealing entry port to allow the admission of fluids, such as saline or image contrast materials, or other materials or devices, into the working channel, for delivering into the patient's bloodstream.

A further advantage of the working channel is that it enables a said guide wire to be inserted therealong to assist in the insertion and positioning of the device in the patient. Thus, a further significant advantage is that the device does not require unusual insertion techniques. It can be inserted relatively straight forwardly by a surgeon or an anaesthetist. For example, where the device is to be used for temporary control of extravasation from the retro-hepatic vena cava, or the hepatic veins, a surgeon or anaesthetist can insert the device quickly from above by inserting it into a major vein in the patient's neck (the lumen of the Internal Jugular Vein is in direct continuity with that of the IVC via the Right Atrium of the heart). Also, the device is not orientation dependant i.e. it cannot be inserted "upside down", since it is basically an inflatable tube.

A wall of the elongate conduit preferably contains, in the thickness thereof, a second channel which is in fluid communication with the inflatable volume of the device, and via which saline or other biologically compatible fluid is injected into the device in order to inflate the device. In this case an input end (for receiving the saline) of the second channel preferably terminates in and is connected to a second self-sealing entry port provided in the same end portion of the elongate conduit as the first entry port. In use, saline is injected via said second self-sealing entry port, along the second channel in the elongate conduit, to inflate the device. The wall(s) of the central elongate conduit may contain one or more further channels in fluid communication with further separate entry ports provided on the same protruding end of the central elongate conduit, for separately carrying saline, contrast imaging fluids or other fluids or devices (e.g. blood or fluid for resuscitation purposes) therealong, to, from or through the device.

The device may be fabricated from one or more physiologically acceptable materials such as various rubber, synthetic rubber, or plastics materials e.g. latex, polyvinyl chloride, polyurethane, polyethylene, Pluronic (RTM), and/or block, and/or co-polymers thereof. For example, the inflatable tube means may be made of latex while the central elongate conduit may conveniently be made of polyurethane.

The device may further include at least one inflatable support strut within the passage means of the device and which may span the inner diameter of the device. The or each said support strut preferably has a hollow interior which is in fluid communication with the enclosed inflatable volume of the device. The or each said support strut provides additional structural integrity to the device when deployed to its at least partially or fully inflated condition. Various configurations of said support strut are possible. Moreover, a plurality of support struts may be provided e.g. two, three, four or more support struts. These support struts are preferably formed and arranged such that, in the inflated state of the device, the struts extend radially outwardly from the central elongate conduit to an annular outer portion of the preferably generally cylindrical form of the inflated device.

The support struts are, in the preferred embodiment, fluidly connected to the inner, saline carrying, channel formed in the wall of the central elongate conduit as above-described, whereby the device is inflated by injecting saline (or another physiologically compatible fluid) through this inner channel in order to inflate the support strut and, consequently, the annular outer portion of the device.

In a further preferred embodiment of the device, the tube forming the device is formed from at least two inflatable semi-circular wall portions, each wall portion having two major longitudinally extending edges which extend substantially parallel to a corresponding edge of an adjacent wall portion; said at least two wall portions is in fluid communication with a support strut, wherein said support strut is formed and arranged to align the respective major edges of the at least two wall portions such that the major edges of adjacent wall portions abut against one another to form a more or less fluid tight seal therebetween and along the extent thereof when the device is in an inflated condition.

Preferably, more than one strut is provided per wall portion. Two, three or four struts can be provided per wall portion. Desirably two struts are provided per wall portion.

It will of course be appreciated that where the tube comprises only two wall portions, then only a single strut which extends between the two wall portions need be provided to ensure that the major edges of adjacent wall portion are maintained in alignment with one another.

Where three, four, five or more wall portions are provided, then each wall portion is provided with a strut, wherein each strut is adjoined to a strut of an adjacent wall portion. In such an arrangement the wall portions may be individually and separately inflatable via said struts, or may be inflated together when said struts are all in common fluid communication with one another.

The inflated device may be non-uniform in outer diameter along the longitudinal length thereof. The required device preferably comprises end portions and a waist portion disposed therebetween, wherein the waist portion has a reduced outer diameter portion extending at least part-way around the circumference of the waist portion, whereby when inflated within a blood/biological fluid conveying vessel, the end and waist portions provide a leak-proof fit between the inner wall (intima) of the vessel and the outer surface of the device, and the reduced diameter portion defines a working cavity between the outer surface of the device and the intima.

Desirably, the reduced outer diameter portion extends longitudinally of the length of inflated device to at least the same extent as the extent to which the reduced outer diameter portion extends around the circumference of the inflated device i.e. the inflated device resembles a "canoe" type shape. Most preferably, the inflated device presents a generally hour-glass shaped side profile, having a relatively narrow waist portion and relatively wide end portions. In this embodiment the wide end portions, when the vessel is in its inflated condition, provide a leak-proof fit with the inner wall (intima) of the blood/biological fluid conveying vessel. The relatively narrow waist portion defines a working space between the outer surface of the device and the damaged portion of the blood/biological fluid conveying vessel, in which working space a surgeon can effect repair to the damaged area.

Furthermore, the hour-glass shaped device may be of particular use within branched vessels such as the Inferior Vena Cava (IVC) which has attached blood vessels such as the hepatic veins. For example, during a liver transplant the hour-glass shaped device may be inserted into the IVC (in a deflated condition) so that one end of the device is positioned above the hepatic veins and the other end is positioned below the hepatic veins. Inflating the device provides a leak tight fit between the ends of the device and the intima of the IVC while maintaining blood-flow through the IVC, and at the same time isolating the hepatic veins from the blood flowing through the IVC. A "cuff" of the hepatic veins may be formed by incising around the hepatic veins at the point of attachment to the IVC thereby allowing removal of the liver and the hepatic veins while maintaining blood-flow through the IVC.

In a modified embodiment, the device incorporates a waist portion which is separately inflatable to the rest of the inflatable volume of the device. By at least partially deflating the separately inflatable waist portion, when the device is in situ, a generally annular working space between the waist portion and the wall of the damaged vessel can be created and/or enlarged.

In a further embodiment of the above-described invention, the device is provided with one or more strategically positioned openings formed and arranged to allow perfusion of blood therethrough from the passage means defined in the inflated device, in a flow direction generally perpendicular to the axis of the device, to branching blood vessels whose inlet ends are located adjacent the openings in the device, when the device is correctly positioned in use thereof. Additionally, for example, the device may be inserted into a vein whereby blood would be permitted to flow from an associated organ, eg a kidney or liver, thus maintaining perfusion while isolating a damaged portion of the vein which can be repaired. The device may have a non-uniform diameter along its length, having a relatively narrow first end portion (neck portion) being provided with said openings, and a relatively wide second portion (body portion).

According to yet another aspect of the invention there is provided a kit of parts comprising an inflatable device as described above, for inserting into a damaged blood or other fluid carrying vessel, and insertion means for facilitating access to the inside of said vessel. The kit-of-parts preferably also includes deployment means for deploying the balloon to its inflated condition.

The insertion means may conveniently comprise a needle for gaining access to a portion of the blood vessel (or other biological fluid carrying which is damaged (or other blood vessel connected vessel) thereto), and a guide wire for inserting into the blood vessel through a bore of the needle. The insertion means preferably further includes sheath means through which the inflatable device may be inserted into the blood vessel. The elongate guide wire is preferably flexible when transverse force is applied to it to allow it to flex or bend without being permanently deformed. The sheath is preferably tubular in form, and is advantageously tapered at one end thereof to facilitate insertion into the lumen of the damaged blood vessel.

The inflatable device is preferably provided with a central elongate conduit, as above described, having a working channel through which the guide wire may be inserted, and a second channel which is in fluid communication with the inflatable volume of the device. The deployment means preferably comprises syringe means for injecting saline, or another biologically compatible fluid, into the inflatable device via said second channel in the elongate conduit.

By means of the above-described kit, the inflatable device can be inserted and deployed in the abdomen of a patient by a surgeon, or alternatively and more conveniently by another doctor outside the operative field (e.g. an anaesthetist).

According to another aspect of the invention use of an inflatable device as above-described, in therapy, is claimed. In particular, use of the above-described inflatable device in the control of extra-vascular haemorrhage is claimed, and especially use of the above-described inflatable device in occluding a damaged portion of the inferior vena cava, or one of the hepatic veins.

According to a further aspect of the invention there is provided a method of controlling extra-vascular haemorrhage comprising inserting the above-described inflatable device, in its deflated condition, into a damaged blood vessel, locating the device adjacent to a damaged portion of the blood vessel, and inflating the device so as to substantially occlude the damaged portion so as to minimise, preferably substantially prevent, leakage of blood from said damaged portion. The damaged blood vessel may, for example, be the inferior vena cava, or one of the hepatic veins. In this case, the insertion step of the method preferably comprises inserting the device in a vein in the neck of the patient.

Alternatively the vessel may be the aorta and the damaged portion thereto may, for example, be a tear in the aorta resulting from traumatic disruption of the aorta or injury to the arch of the aorta. In this case, access via the arterial system is necessary and thus the insertion step may conveniently comprise inserting the device into the patient from below, via the femoral artery.

Further applications of the above-described inflatable device are also possible. In particular, the device may find application in the field of stenting. In conventional stenting blood flow is temporarily blocked when the balloon carrying the (collapsed) stent is inflated. It is well known that inserting a stent into a vessel, such as the Thoracic Aorta, has a particular problem associated therewith in that both the balloon and the stent migrate in the direction of the blood-flow through the vessel as the balloon is inflated. The migration is a result of the balloon acting effectively as a piston within the vessel whereby the blood acts directly thereagainst. This problem of stent and balloon migration is undesirable, especially where each stent is relatively expensive and in some cases are custom-made for a particular patient and surgical procedure. In contrast, the tubular form of the inflated device of the present invention will allow blood to flow relatively unrestricted therethrough during expansion of the stent. Thus, in accordance with another aspect of the invention there is provided a method of inserting and deploying a stent in a blood vessel, the method comprising the steps of: locating an expandable generally tubular stent, in a collapsed state thereof, on the above-described inflatable device so that the stent is coaxial with and circumferentially surrounds the inflatable device; inserting the inflatable device, in its deflated condition, and with the stent located thereon, into the blood vessel to be stented and locating the device in the desired portion thereof; and inflating the device so as to deploy the stent to an expanded condition in which it supports the wall of the blood vessel.

Preferred embodiments of the invention will now be described by way of example only and with reference to the accompanying drawings in which:
Fig. 1 is a plan view of a device according to one embodiment of the invention, being inserted into a blood vessel;
Fig. 2 is a side view of the device being inserted into the blood vessel as in Fig. 1, the blood vessel being shown in cross-section so as to reveal the portion of the device inserted therein;
Fig. 3 shows the device or Figs. 1 and 2, deployed (i.e. in its inflated state) in the blood vessel which is again shown in cross-section;
Fig. 4 is an end cross-section of the inflated device of Fig. 3;
Fig. 5 is a longitudinal sectional view of an inflated device according to another embodiment of the invention, shown located in a blood vessel;
Fig. 6 is a longitudinal sectional view of an inflated device according to a further embodiment of the invention;
Fig. 7 is a side view of a device according to a yet further embodiment of the invention, having a reduced diameter end portion with openings therein;
Fig. 8 illustrates blood-flow pathways through the device of Fig. 7;
Fig. 9 shows the device of Figs. 7 and 8, in an inflated condition, in situ in a blood vessel with a leaking aneurysm, the blood vessel being shown in cross-section so as to reveal the device;
Fig. 10 is a side view of an inflated device according to a yet further embodiment of the present invention;
Fig. 11 is a plan view looking down onto the device of Fig. 10;
Fig. 12 is a schematic side view of the device of Figs 10 and 11 located within the Inferior Vena Cava adjacent the hepatic veins and liver;
Fig. 13 shows the liver of Fig. 12 with a cuff cut from the Inferior Vena Cava;
Fig. 14A and Fig. 14B shows a section of a further preferred embodiment of the device in deflated and inflated conditions respectively where the tube is formed from three separate wall portions adjoined by struts.

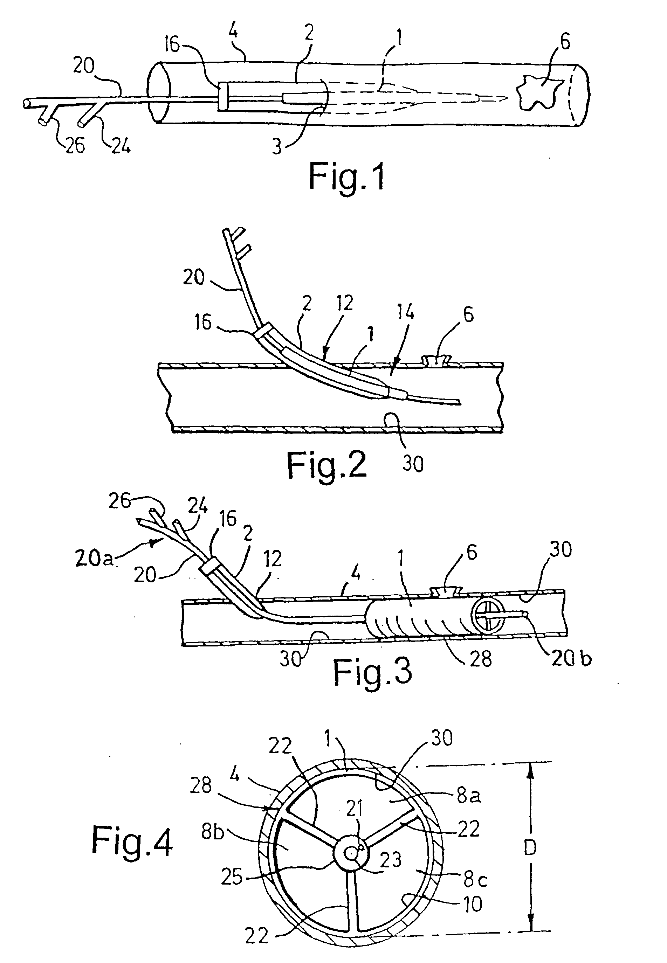

An inflatable device 1 according to one embodiment of the present invention is shown in Figs. 1-4. Figs. 1 and 2 show the device in its deflated condition being inserted, via a sheath 2 provided for use with the device 1, into a blood vessel 4 which has a damaged portion 6. The device 1 comprises a balloon which, when inflated (as shown in Fig. 3), defines a generally tubular member of substantially cylindrical open-ended form, a passage 8 being defined therethrough by an inner wall 10 of the device 1, as shown most clearly in Fig 4.

As illustrated in Figs. 1 & 2, to facilitate insertion of the device 1 into a blood vessel 4 a sheath 2 is provided for insertion into the blood vessel 4. In Figs. 1-3 the sheath 2 is shown as transparent in order to reveal the device 1 being pushed therethrough into the lumen of the blood vessel. A first, slightly tapered, end 14 of the sheath 2 is inserted into the blood vessel 4 and a second end of the sheath 2 protrudes from the blood vessel 4. The second end 16 of the sheath 2 is self-sealing around a central elongate tube 20 which extends through, and protrudes rearwardly from, the inflatable device 1. The insertion procedure is described in further detail herebelow.

As seen most clearly from Figs. 3 & 4, the central tube 20 extends axially through the passage 8 and protrudes from each end of the device. The central tube 20 is hollow, the hollow interior acting as a working channel 23 for delivery of fluids into the damaged blood vessel, beyond the damaged portion 6 thereof. A smaller separate channel 21 which is in fluid communication with the inflatable volume of the device 1, is incorporated within the thickness of the wall 25 of the central tube 20. Three elongate inflatable struts 22 branch outwardly from the working channel 23 to the inner wall 10 of the device 1, effectively dividing the passage 8 into three separate passage sections 8a, 8b, 8c, and are in fluid communication with the central tube 20 which is provided at a first end 20a thereof with a self-sealing side entry port 24 for the injection of saline (or other physiologically compatible fluid) into this smaller channel 21 to effect inflation of the device 1. A second self-sealing side entry port 26 is also provided at the first end 20a of the central tube 20 for injection of contrast fluids or other materials into the blood vessel 4, via the working channel 23, for imaging or other similar purposes. The first and second entry ports 24, 26 are connected to one end of the smaller channel 21 and working channel 23 respectively. As shown in Figs. 2 and 3 there is a relatively long length (approx. one metre or so) of central tube 20 between the inflatable portion of the device and the first end 20a of the central tube having the side entry ports 24, 26. The other end 20b of the central tube protrudes only a short way (a few centimetres) beyond the inflatable tube portion of the device.

A guide wire (not shown) is used to facilitate insertion and positioning of the device 1 in the blood vessel 4. The guide wire is formed from a physiologically compatible alloy of metal such as known medical quality steels (stainless steels). In use, the device 1 is inserted as follows. Firstly, a small incision is made in the patient's skin in the region of, but not into, a blood vessel (e.g. vein or artery) into which the device 1 is to be inserted. This blood vessel could be the vessel having the damaged portion, or a vessel connected to the damaged one and through which access to the damaged vessel can therefore be obtained. For example, when the damaged vein is the inferior vena cava, access thereto can be obtained by inserting the device 1 into the internal jugular vein in the patient's neck, connected to the superior vena cava. A needle (hypodermic) is then used to locate the blood vessel itself, by inserting the needle thereinto (via the incision in the skin). A guide wire (not shown) is then threaded through the needle, into the blood vessel. The needle is then removed. A dilator (not shown) can then be used to enlarge the opening into the blood vessel (created by the needle) and, after removing the dilator, the tapered end 14 of the sheath 2 is inserted into the aperture. The working channel of the elongate tube member is then threaded over the guide wire and the device 1 is pushed (manually) therealong, through the sheath 2. The guide wire can be removed at this point (and in any case is removed prior to inflation- of the device 1). The device 1 is continued to be pushed along the lumin of the blood vessel, through the sheath 2, with the long end of the central tube 20 trailing behind the deflated device 1, until the device 1 is located adjacent to the damaged portion 6 in the wall of the blood vessel. The device 1 is then inflated by the injection of saline, so as to occlude the damaged portion 6.

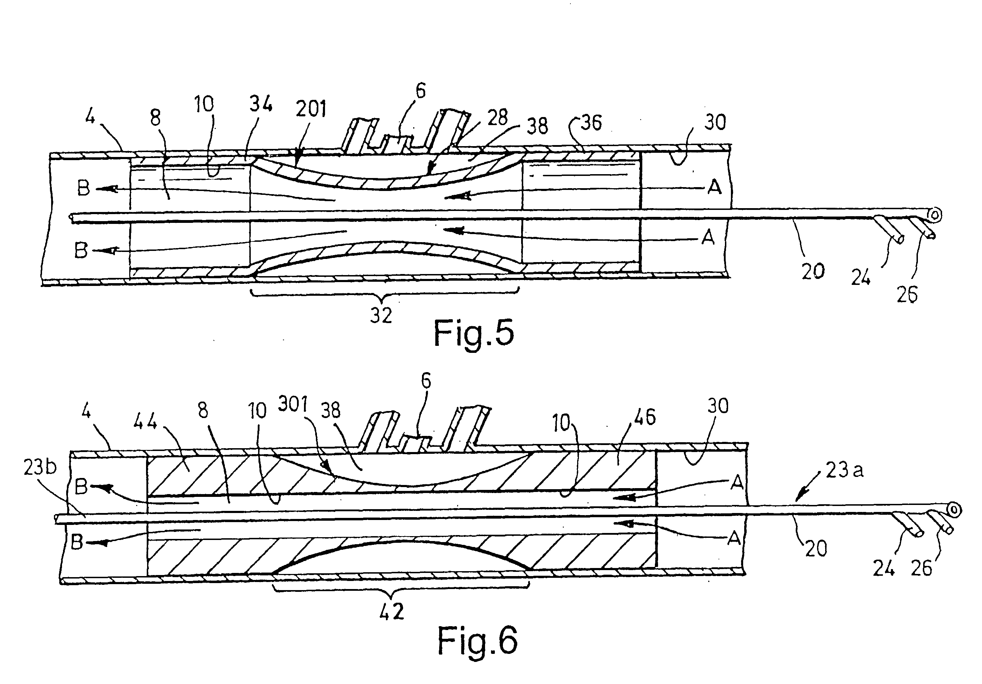

The device 1 in an inflated condition, as shown in Fig. 3, comprises a generally cylindrical member which is supported at least in part by the inflatable support struts 22. As aforedescribed, the support struts 22 are in fluid communication with the inflatable volume of the device 1 and receive saline solution (or another physiologically acceptable fluid which is injected into the inflatable volume of the device) from the inflation channel 21 in the central tube 20. When inflated, the outer wall 28 of the device 1 is pressed flush against the inner wall (intima) 30 of the blood vessel 4, to form a leak-free seal between the device 1 and the inner wall of the vessel 4. With the device in its inflated condition, as shown in Figs.3 and 4, blood flows through the passage 8 (see arrows A to B, Figs. 5, 6) relatively unhindered, while the damaged portion 4 of the vessel 2 is repaired by a surgeon. (Blood may alternatively flow in the direction of B to A in Figs. 5 and 6). The diameter of the passage 8 is sufficiently large to ensure that blood flows through the device 1 relatively unhindered i.e. at or approaching normal flow rates in the vessel. This is achieved by designing the diameter of the passage means to be in the range of 80 to 90% of the outer diameter D of the device.

Preferably, at least those surfaces of the device which will be in contact with blood are formed from and/or coated with an anticoagulant material e.g. heparin. One of the benefits of having anticoagulant materials present on the surface of the device is that the device may thus be left in situ for several hours, during which time the blood/biological fluid conveying vessel can be repaired by a surgeon and the patient transported if necessary.

A modified device 201 according to a second embodiment of the present invention is shown in Fig. 5, in which like parts to the device in Figs. 1-4 are referenced by like reference numerals. The modified device 201 has a relatively narrow diameter waist portion 32 located between two relatively wide diameter tubular end portions 34, 36 in the inflated state of the device. The inflated device thus has a substantially hour-glass profile, as shown in Fig. 5. In use of the device, the two end portions 34, 36 are located to either side of the damaged portion 6 of the blood vessel 4 so that the waist section 32 is located at the damaged portion 6. With the device 201 inflated, the end portions 34, 36 provide a leak-proof seal between the device 201 and the inner wall of the blood vessel 4 thereby preventing blood leakage out of the vessel 4 at the damaged portion 6 thereof. This allows a surgeon to repair the damaged portion 6 of the vessel 4. Furthermore, as a result of the relatively reduced diameter of the waist portion 32, an approximately annular space 38 is created between the inner (damaged) wall 30 of the blood vessel and the outer surface 28 of the waisted portion 32 of the device. This annular space 38 allows a surgeon to work to repair the damaged portion 6 with a reduced risk of compromising the integrity of the device 201 by damage thereto by, for example, a surgical instrument (e.g. knife, needles or scissors) used by the surgeon during repair of the blood vessel 4.

A device 301 according to another modified embodiment of the present invention comprises a separately inflatable waist section 42 between first 44 and second 46 inflatable end portions, as shown in Fig. 6. The device 301 in a collapsed (non-inflated condition) is inserted into a blood vessel 4 to be repaired, and the first and second end portions 44, 46 are inflated by injecting saline into the inner volumes thereof. In this embodiment the central tube 20 incorporates two separate channels in the thickness of the tube wall 25, one for delivering saline to the end portions 44,46 and the other for delivering saline to the waist section 42. A first entry port 24 at the input end of the tube 20 is connected to a first one 50 of these two channels which feeds saline to one or more support struts 22 which are in fluid communication with the first and second end portions 44 and 46 of the device. A second one 52 of these two channels connects a second entry port 26 in the tube 20 to the separately inflatable waist section 42, via a separate one of the support struts 22.

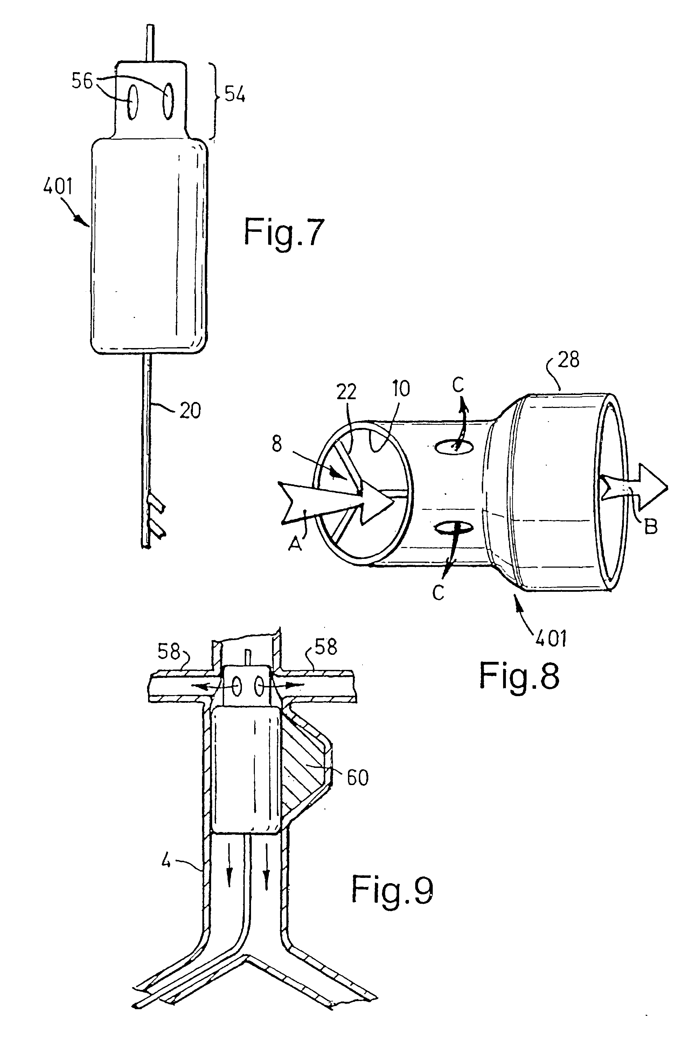

Where the devices of Figs.1-6 are intended for use in occluding a damaged portion of the vena cava or hepatic veins, the length L of the inflatable tubular portion of the device will typically be in the range of 13 to 17cm long, while the central tube 20 will typically be approximately 1 metre long. The diameter of the device, when collapsed, would typically be 3 to 4mm and, when inflated, about 2 to 2.5cm. However, it will be appreciated that the dimensions may be different for where the device is intended for use in different blood vessels. E.g. for use in the aorta to, for example, occlude a diseased portion thereof, the inflatable tubular portion is likely to be approximately 7cm long and 4cm in diameter (when inflated). Figs. 7-9 illustrate a modified version of the device of Fig. 3, for achieving control of extravasation while preventing ichaema (reduction in blood flow) to other body parts. (Again, like parts to Figs. 1-4 are referenced by like reference numerals). This embodiment would be used for cases where a body part is supplied with blood via a blood vessel which branches off from the damaged blood vessel 4 (which may, for example, be a main blood vessel which is damaged or injured very close to, or at the branching point, from which the body part derives its blood supply). In a case like this, the normal placement of a device such as shown in Figs. 3, 5 or 6 may occlude the branching point of the vessel supplying the body part. This would occur if for example the inferior vena cava was damaged close to the hepatic or renal veins, or where there was an aneurysm 60 below the renal arteries 58, as shown in Fig.9. In either of these cases, if blood flow to these branching vessel(s) was interrupted for any significant period of time such as would be required to repair the damaged blood vessel, then the function and vitality of the liver and kidneys respectively would be considerably compromised, perhaps fatally so.

Thus according to the modified embodiment of Figs.7-9, the inflatable device 401 is provided with a relatively narrow neck portion 54 with openings 56 therein which allow blood flowing through the passage 8 in the device 401 (in use thereof) to enter from, or exit to, blood vessels 58 branching off from the main (damaged) blood vessel 4. As seen most clearly from Figs. 8, the openings 56 are positioned to allow blood to flow out of the passage 8, generally perpendicularly to normal blood flow through the passage 8. As explained above, this device 401 is particularly suitable for use when repairing a leaking aneurysm 60 (Fig. 9) while maintaining a blood supply to, for example the renal arteries 58. This is of significant benefit as the kidneys (or other organs) can thus be supplied with blood throughout the period in which the surgeon effects a repair to the main blood vessel 4, or where transfer of the patient to another location is required, or where there is any delay while an expert is in transit to the patient's location, thus controlling the leak and simultaneously allowing renal perfusion.

The relative positions of the openings 56 are chosen to suit the particular purpose i.e. the positions will generally be dependant upon which organs are to be maintained with a blood supply. Where only one organ is to be supplied, then a device 401 with only one opening may be used. As will be appreciated the blood supply required by various organs is different and so too is the vessel size supplying blood to the organ(s), therefore the openings may correspondingly be of different sizes to accommodate various blood flow requirements.

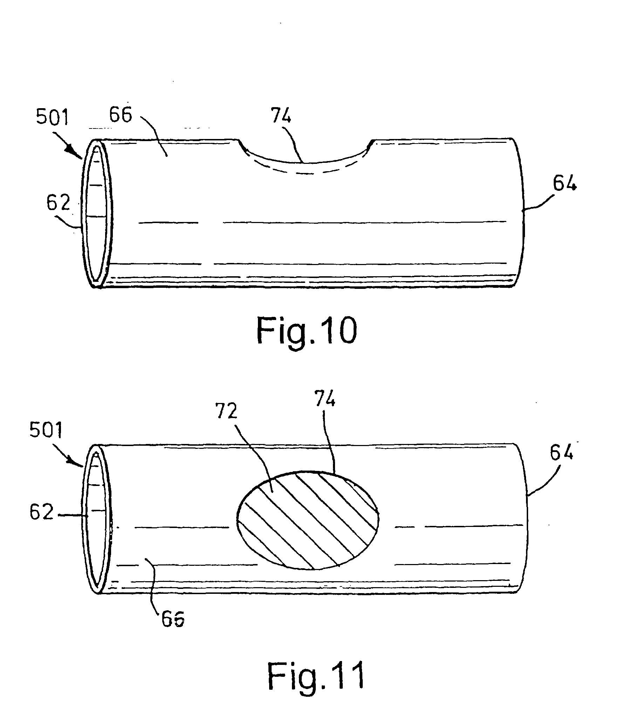

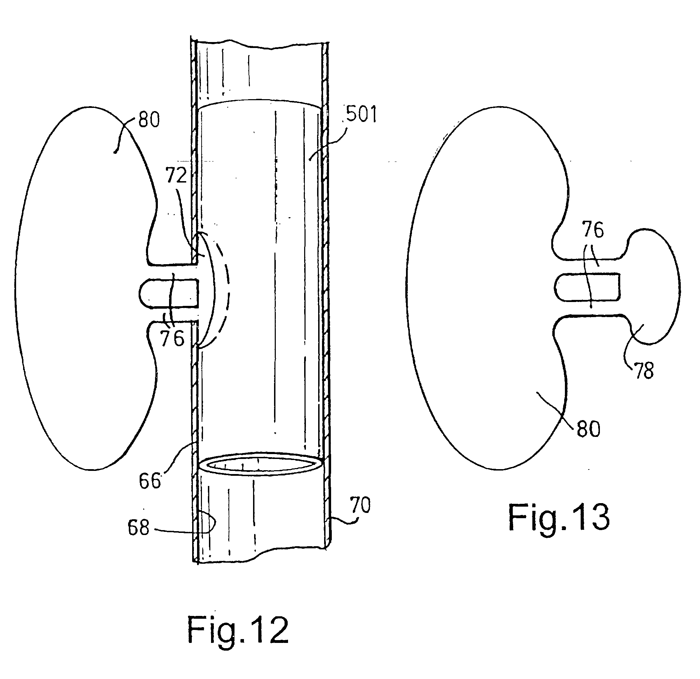

Figs 10 and 11 show a yet further modified embodiment of a device according to the present invention as indicated generally by the reference numeral 501. The device 501 is cylindrical, being equal diameter as it extends between first 62 and second 64 ends. The device 501 has an outer wall 66 which is in leak-proof contact with the initima 68 Fig 12 of the Inferior Vena Cava 70.

The device 501 has an oval shaped depression 72 (see shaded region in Fig 11) formed mid-way between the first and second ends 62, 64. The depression 72 has an outer edge 74 which is in leak-proof contact with the initima 68 when the device 501 is in an inflated condition. The depression 72 forms a working area for a surgeon to operate. As indicated in Fig 12, the device 501 when inserted into the IVC and locating the depression over the hepatic veins 76, allows a surgeon to incise about the hepatic veins to form a cuff 78 (Fig 13) and thereby effect removal of the liver 80. The device 501 as hereinbefore described is particularly useful in allowing a surgeon to form the cuff 78 during organ transplantations such as for the liver (Figs 12 and 13) and other organs such as the kidneys.

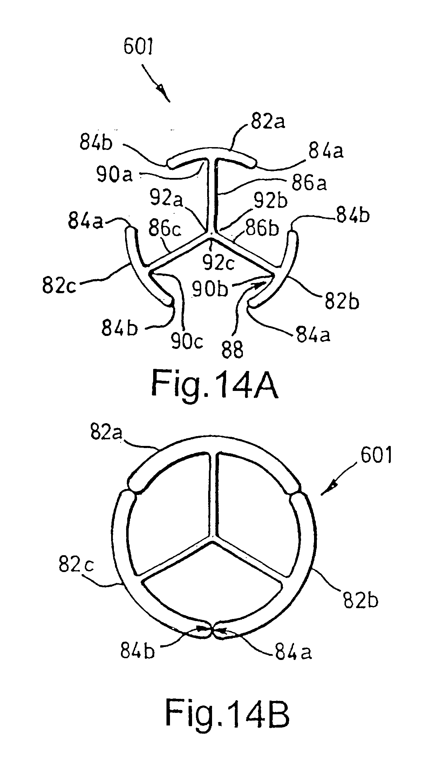

In a further preferred embodiment as shown in Figs 14A and 14B, the device 601 is formed from three inflatable semi-circular section wall portions 82a,b,c arranged to extend radially from the centre of the device 601. Each wall portion 82a,b,c has two major sides 84a,b which extend along the length of the device 601.

An inflatable strut 86a,b,c extends inwardly from an inner surface 88 of each of the walls 82a,b,c. Each strut 86a,b,c is in fluid communication with the respective wall portion 82a,b,c at a first end 90a,b,c of each strut 86a,b,c, whereas all three struts 86a,b,c are also in fluid communication with one another at second ends 92a,b,c thereof where the struts 86a,b,c and conjoined to one another. In a deflated state as shown in Fig 14A, the major edges 84a,b of the wall portions 82a,b,c are spaced apart from one another. When in an inflated state (Fig 14B) the major edges 84a,b of adjacent wall portions 84a,b,c are in full contact with one another so that fluid may pass through the device 601 without leakage between the major edges 84a,b.

It will be appreciated that various modifications to the above-described embodiments are possible. For example, the guide wire may be formed from a so-called "memory" material which material adopts a pre-determined shape or configuration where the device is in situ within the lumen of the damaged vessel, or other desired location.

Furthermore saline could be fed to the inflatable volume of any of the above-described devices by a tube connected directly to the generally cylindrical (or hour-glass shaped) annular member of the device, instead of incorporating a saline channel in the wall of the central tube 20. In this case there may be no central tube 20 provided.

It will further be appreciated that as well as being applicable for use in control of extravasation from, for example, the IVC or hepatic veins, this being most commonly required in trauma cases, the above-described device is also suitable for management of non-trauma diseases such as aortor-cava fistula (i.e. direct communication between aorta an IVC due to aneurysm etc), where the device could be used to retain blood flows in their respective lumina.

Furthermore, and as already mentioned above, the device may be used in the deployment of a stent in a blood vessel. The stent may simply be threaded over the inflatable tubular portion of the device 1 of Figs.1-4 above, for example, so that the stent is coaxial with and circumferentially surrounds the inflatable device. The inflatable device is then inserted into the desired blood vessel in the same manner as described above, and then inflated so as to deploy the stent to its expanded state. Again, the advantage of blood being able to flow through the inflatable tubular portion of the device is that normal or near normal blood flow is maintained in the patient while the stent is deployed to its expanded state in which it supports the blood vessel wall.

A further modification that may be made to the device of the present invention, particularly where the device is to be used for a specific type of procedure is where the device is to be used for stenting, then the device may be provided with radiological markers thereon. To facilitate visualising the position and location of the device within a vessel (such as the Aorta) during X-Ray screening, the balloon or inflatable tube portion may be provided with radiological markers at each end. Additionally or alternatively the radiological markers may be located or scaled so as to facilitate assessment of the - internal diameter of the vessel.

## Claims

1. An inflatable device for inserting, in a substantially deflated state, into a blood or other biological fluid carrying vessel of a human or animal, the device having an inflated state in which it defines an elongate open-ended tube means having an inlet end and an outlet end, said tube having a first collapsible outer wall formed and arranged to provide, in said inflated state, a close fit around the whole of the intima of the vessel in use of the device, and a second collapsible inner wall closely spaced apart from said first outer wall in said inflated state, said second inner wall defining a substantially uninterrupted passage means through the device through which blood or other biological fluid may flow, in use of the device, said first outer wall and said second inner wall defining an inflatable volume, whereby in use of the device in an inflated state, blood or other biological fluid may pass through the device without leaking past or around the outside of the device, and without the flow thereof therethrough being substantially interrupted.

2. A device as claimed in claim 1 wherein the elongate tube means defined by the inflated device comprises at least one passage, defined between said inlet end and said outlet end, through the longitudinal length of the device.

3. A device as claimed in claim 1 or claim 2 wherein the diameter of said first inner wall is greater than 50% of the diameter of the second outer wall of the inflated device.

4. A device as claimed in claims 1 or 2 wherein the diameter of said first inner wall is in the range of 90 to 95% of the diameter of the second outer wall of the inflated device.

5. A device as claimed in any one of claims 1 to 4, wherein the inflatable volume is in fluid communication with an inflation channel disposed inwardly of the inner wall.

6. A device as claimed in any one of claims 1 to 5 wherein the device has a cross-section which is generally circular or elliptical when inflated.

7. A device as claimed in any one of claims 1 to 6 wherein the device has an irregular cross-section when inflated, according to the particular shape or configuration of the damaged blood or biological fluid carrying vessel in which the device is to be used.

8. A device as claimed in any one of claims 1 to 7 wherein in use of the device in a vessel having an injury area, the device has a length which extends to at least the same length as said injury area in a said vessel.

9. A device as claimed in any one of claims 1 to 8 wherein the device has a working channel or conduit extending at least part-way through the device wherein the working channel or conduit is formed and arranged for receiving therein at least one of the group comprising: a fluid, a device and a guide-wire.

10. A device as claimed in claim 9 wherein the working channel or conduit is the hollow interior of an elongate conduit centrally positioned within the passage means.

11. A device as claimed in claim 10 wherein the elongate conduit is integrally formed with the inflatable device.

12. A device as claimed in claims 10 or 11 wherein the elongate conduit is fixed to the device at at least one point therealong, with opposite end portions of the elongate conduit protruding from respective opposite ends of the tube means defined by the inflated device.

13. A device as claimed in claim 12 wherein a first one of said protruding end portions is relatively long while the second end portion_protrudes only a very short length beyond the tube means forming the inflated device.

14. A device as claimed in claim 13 wherein said first protruding end portion is provided with a first self-sealing entry port to allow the admission of fluids materials or devices, into the working channel, for delivery into a patient's bloodstream.

15. A device as claimed in any one of claims 1 to 14 wherein the device is formed and arranged to slide over a guide wire to assist in the insertion and positioning of the device.

16. A device as claimed in any one of claims 10 to 15 wherein a wall forming the elongate conduit contains, in the thickness thereof, an inflation channel in fluid communication with the inflatable volume of the device, and through which a biologically compatible fluid is injected into the device in order to inflate the device.

17. A device as claimed in claim 16 wherein an input end of the inflation channel terminates in and is connected to a second self-sealing entry port provided in the same end portion of the elongate conduit as the first entry port.

18. A device as claimed in claims 16 or 17 wherein one or more walls forming said central elongate conduit contain one or more further channels in fluid communication with further separate entry ports provided on the same protruding end of the central elongate conduit, for separately carrying one or more of a biologically compatible fluid, contrast imaging fluids and other fluids or devices therealong, to, from or through the device.

19. A device as claimed in any one of claims 1 to 18 wherein the device has at least one inflatable support strut within the passage means, said inflatable support strut being in fluid communication with said inflatable volume.

20. A device as claimed in claim 19 wherein the at least one strut spans the inner diameter of the device.

21. A device as claimed in claim 19 or 20 wherein two, three, four or more support struts are provided.

22. A device as claimed in claim 19 or 20 when dependant on any one of claims 10 to 18 wherein the at least one support strut is formed and arranged such that, in the inflated state of the device, the strut extends radially outwardly from the central elongate conduit to the annular outer portion of the inflated device, defined between said first inner wall and said second outer wall.

23. A device as claimed in any one of claims 19 to 22 when dependant on any one of claims 16 to 18 wherein the at least one support strut is in fluid communication with the inner channel formed in the wall of the central elongate conduit.

24. A device as claimed in any one of claims 1 to 23 wherein the inflated device is non-uniform in outer diameter along the longitudinal length thereof.

25. A device as claimed in any one of claims 1 to 24 wherein the inflated device presents a generally hour-glass shaped side profile, having a relatively narrow waist portion and relatively wide end portions.

26. A device as claimed in claim 25 wherein the device in its inflated condition, provides a leak-proof fit with the intima of the blood/biological fluid conveying vessel and the waist portion defines a working space between the outer surface of the device and the intima of the blood/biological fluid conveying vessel.

27. A device as claimed in either of claims 25 and 26 wherein the waist portion is separately inflatable from the rest of the inflatable volume of the device.

28. A device as claimed in any one of claims 1 to 27 wherein the device is provided with one or more openings through the side walls of said tube and which are formed and arranged to allow perfusion of blood therethrough from the passage means in a flow direction generally perpendicular to the axis of the device when said openings are located at branching points within a branched vessel.

29. A device as claimed in claim 28 wherein the device has a non-uniform diameter along its length, having a relatively narrow first end portion provided with said openings, and a relatively wide second portion.

30. A device as claimed in claims 24 to 29 wherein the device has a waist portion disposed between first and second ends of the device, wherein the waist portion has a reduced outer diameter portion extending at least part-way around the outer circumference of the device and defines a working area between the intima of a said vessel and the inflated device.

31. A device as claimed in claim 30 wherein the reduced outer diameter portion has a longitudinal extent at least as great as its extent about the circumference of the device.

32. A device as claimed in any one of claims 19 to 31 where the tube forming the device is formed from at least two inflatable semi-circular wall portions, each wall portion having two major longitudinally extending edges which extend substantially parallel to a corresponding edge of an adjacent wall portion; said at least two wall portions are in fluid communication with a said support strut, wherein said support strut is formed and arranged to align the respective major edges of the at least two wall portions such that the major edges of adjacent wall portions abut against one another to form a more or less fluid tight seal therebetween and along the extent thereof when the device is in an inflated condition.

33. A device as claimed in any one of claims 1 to 32 wherein the device is fabricated from one or more physiologically acceptable materials selected from the group including rubber, synthetic rubber, and plastics materials, polyurethane, polyethylene, Pluronic (RTM), and/or block, and/or co-polymers thereof.

34. A kit of parts comprising an inflatable device as claimed in any one of claims 1 to 33 for inserting into a blood or other fluid carrying vessel, and insertion means for facilitating access to the inside of said vessel.

35. A kit-of-parts as claimed in claim 34 which includes deployment means for deploying the device to its inflated condition.

## Patentansprüche

1. Aufblasbare Vorrichtung zum Einsetzen, in einem im wesentlichen entleerten Zustand, in ein Blut oder ein anderes biologisches fluidführendes Gefäß eines Menschen oder eines Tieres, wobei die Vorrichtung im aufgeblasenen Zustand, in dem sie ein längliches, offene Enden aufweisendes Rohr mit einem Einlassende und einem Auslassende bildet, wobei das Rohr mit einer ersten, zusammenfallbaren äußeren Wand versehen ist, die ausgebildet und angeordnet ist, um beim Gebrauch der Vorrichtung im genannten aufgeblasenen Zustand eine enge Anpassung an die gesamte Intima des Gefäßes zu erreichen, und das Rohr mit einer zweiten, zusammenfallbaren inneren Wand ausgerüstet ist, die sich im genannten aufgeblasenen Zustand im kurzen Abstand von der ersten, äußeren Wand befindet, wobei die zweite, innere Wand einen im wesentlichen nicht unterbrochenen Durchgang durch die Vorrichtung bildet, durch den beim Gebrauch der Vorrichtung Blut oder ein anderes biologisches Fluid strömen kann, und wobei die genannte erste, äußere Wand und die genannte zweite, innere Wand ein aufblasbares Volumen bilden, wodurch beim Gebrauch der Vorrichtung in einem aufgeblasenen Zustand Blut oder ein anderes biologisches Fluid durch die Vorrichtung hindurchströmen kann, ohne an der Außenseite der Vorrichtung vorbei und um sie herum auszutreten und ohne daß der Strom dieser hindurchtretenden Stoffe wesentlich unterbrochen wird.

2. Vorrichtung nach Anspruch 1, worin das von der aufgeblasenen Vorrichtung gebildete längliche Rohr über die Länge der Vorrichtung mindestens einen Durchgang aufweist, der zwischen dem genannten Einlassende und dem genannten Auslassende ausgebildet ist.

3. Vorrichtung nach Anspruch 1 oder 2, worin der Durchmesser der ersten, äußeren Wand größer als 50 % des Durchmessers der zweiten, inneren Wand der aufgeblasenen Vorrichtung ist.

4. Vorrichtung nach Anspruch 1 oder 2, worin der Durchmesser der zweiten, inneren Wand im Bereich von 90 bis 95 % des Durchmessers der ersten, äußeren Wand der aufgeblasenen Vorrichtung liegt.

5. Vorrichtung nach einem der Ansprüche 1 bis 4, worin das aufblasbare Volumen in Fluidverbindung mit einem Aufblaskanal steht, der von der Innenwand nach innen angeordnet ist.

6. Vorrichtung nach einem der Ansprüche 1 bis 5, worin die Vorrichtung, wenn sie aufgeblasen ist, einen Querschnitt aufweist, der im allgemeinen kreisförmig oder elliptisch ist.

7. Vorrichtung nach einem der Ansprüche 1 bis 6, worin die Vorrichtung, wenn sie aufgeblasen ist, einen unregelmäßigen Querschnitt aufweist, entsprechend der besonderen Gestalt oder Konfiguration des beschädigten, Blut oder ein biologisches Fluid führenden Gefäßes, in dem die Vorrichtung benutzt werden soll.

8. Vorrichtung nach einem der Ansprüche 1 bis 7, worin beim Gebrauch der Vorrichtung in einem Gefäß mit einem verletzten Bereich die Vorrichtung eine Länge aufweist, die sich in dem Gefäß über mindestens die gleiche Länge wie der verletzte Bereich erstreckt.

9. Vorrichtung nach einem der Ansprüche 1 bis 8, worin die Vorrichtung einen Arbeitskanal oder eine Arbeitsleitung aufweist, die sich über mindestens teilweise durch die Vorrichtung hindurch erstreckt, wobei der Arbeitskanal oder die Arbeitsleitung ausgebildet und angeordnet ist, um mindestens einen der Gegenstände Fluid, Vorrichtung und Führungsdraht aufzunehmen.

10. Vorrichtung nach Anspruch 9, worin der Arbeitskanal oder die Arbeitsleitung das hohle Innere einer länglichen Leitung ist, die innerhalb des Durchgangs zentral angeordnet ist.

11. Vorrichtung nach Anspruch 10, worin die längliche Leitung einstückig mit der aufblasbaren Vorrichtung ausgebildet ist.

12. Vorrichtung nach Anspruch 10 oder 11, worin die längliche Leitung an mindestens einem Punkt ihrer Länge an der Vorrichtung befestigt ist, wobei entgegengesetzte Endabschnitte der länglichen Leitung von entsprechenden Enden des durch die aufgeblasene Vorrichtung gebildeten Rohrs vorstehen.

13. Vorrichtung nach Anspruch 12, worin ein erster der vorstehenden Endabschnitte relativ lang ist, während der zweite Endabschnitt nur über eine sehr kurze Länge über das die aufgeblasene Vorrichtung bildende Rohr hinausragt.

14. Vorrichtung nach Anspruch 13, worin der genannte erste vorragende Endabschnitt mit einer ersten selbstverschließenden Eingangsöffnung versehen ist, um für die Abgabe in den Blutstrom eines Patienten den Zugang von Fluiden, Materialien oder Vorrichtungen in den Arbeitskanal zu ermöglichen.

15. Vorrichtung nach einem der Ansprüche 1 bis 14, worin die Vorrichtung ausgebildet und angeordnet ist, um über einen Führungsdraht zu gleiten und das Einsetzen und Positionieren der Vorrichtung zu unterstützen.

16. Vorrichtung nach einem der Ansprüche 10 bis 15, worin eine die längliche Leitung bildende Wand in ihrer Dicke einen Aufblaskanal in Fluidverbindung mit dem aufblasbaren Volumen der Vorrichtung enthält und durch diesen hindurch ein biologisch verträgliches Fluid in die Vorrichtung eingespritzt wird, um diese aufzublasen.

17. Vorrichtung nach Anspruch 16, worin ein Eingabeende des Aufblaskanals in einer zweiten selbstverschließenden Eingangsöffnung endet und damit verbunden ist, das im gleichen Endabschnitt der länglichen Leitung wie die erste Eingangsöffnung vorgesehen ist.

18. Vorrichtung nach Anspruch 16 oder 17, worin eine oder mehrere Wände, welche die zentrale längliche Leitung bilden, einen oder mehrere Kanäle in Fluidverbindung mit weiteren getrennten Eingangsöffnungen enthalten, die am gleichen vorstehenden Ende der zentralen länglichen Leitung vorgesehen sind, um dort entlang eine oder mehrere biologisch verträgliche Fluide, Kontrastbildfluide und andere Fluide oder Vorrichtungen getrennt zu der Vorrichtung, von der Vorrichtung weg oder durch die Vorrichtung hindurch zu bringen.

19. Vorrichtung nach einem der Ansprüche 1 bis 18, worin die Vorrichtung innerhalb des Durchgangs mindestens eine aufblasbare Tragstütze aufweist, die in Fluidverbindung mit dem genannten aufblasbaren Volumen steht.

20. Vorrichtung nach Anspruch 19, worin mindestens eine Stütze den Innendurchmesser der Vorrichtung überspannt.

21. Vorrichtung nach Anspruch 19 oder 20, worin zwei, drei, vier oder mehr Tragstützen vorgesehen sind.

22. Vorrichtung nach Anspruch 19 oder 20, falls sie von einem der Ansprüche 10 bis 18 abhängig sind, worin die mindestens eine Tragstütze derart ausgebildet und angeordnet ist, daß sie sich im aufgeblasenen Zustand der Vorrichtung von der zentralen länglichen Leitung radial nach außen zu dem ringförmigen Außenabschnitt der aufgeblasenen Vorrichtung erstreckt, die zwischen der genannten ersten, äußeren Wand und der genannten zweiten, inneren Wand ausgebildet ist.

23. Vorrichtung nach einem der Ansprüche 19 bis 22, falls sie von einem der Ansprüche 16 bis 18 abhängig sind, worin mindestens eine Tragstütze in Fluidverbindung mit dem Innenkanal steht, welcher in der Wand der zentralen länglichen Leitung ausgebildet ist.

24. Vorrichtung nach einem der Ansprüche 1 bis 23, worin die aufgeblasene Vorrichtung über ihre Länge in ihrem Außendurchmesser uneinheitlich ist.

25. Vorrichtung nach einem der Ansprüche 1 bis 24, worin die aufgeblasene Vorrichtung ein im allgemeinen sanduhrförmiges Seitenprofil mit einem relativ engen Mittelteilabschnitt und relativ breiten Endabschnitten aufweist.

26. Vorrichtung nach Anspruch 25, worin die Vorrichtung in ihrem aufgeblasenen Zustand eine dichte Passung gegenüber der Intima des Blut oder ein biologisches Fluid führenden Gefäßes ergibt und der Mittelteilabschnitt einen Arbeitsraum zwischen der Außenoberfläche der Vorrichtung und der Intima des Blut oder ein biologisches Fluid führenden Gefäßes bildet.

27. Vorrichtung nach Anspruch 25 oder 26, worin der Mittelteilabschnitt des aufblasbaren Volumens der Vorrichtung getrennt vom Rest dieses Volumens aufblasbar ist.

28. Vorrichtung nach einem der Ansprüche 1 bis 27, worin die Vorrichtung mit einer oder mehreren Öffnungen durch die Seitenwände des genannten Rohrs hindurch versehen ist, die ausgebildet und angeordnet sind, um ein Hindurchdringen von Blut von dem Durchgang aus in einer Strömungsrichtung, die im allgemeinen senkrecht zur Achse der Vorrichtung verläuft, zu ermöglichen, wenn die genannten Öffnungen an Verzweigungspunkten innerhalb eines verzweigten Gefäßes angeordnet sind.

29. Vorrichtung nach Anspruch 28, worin die Vorrichtung über ihrer Länge einen uneinheitlichen Durchmesser aufweist, wobei ein relativ enger erster Endabschnitt mit den genannten Öffnungen und ein relativ breiter zweiter Abschnitt vorliegen.

30. Vorrichtung nach den Ansprüchen 24 bis 29, worin die Vorrichtung einen Mittelteilabschnitt aufweist, der sich zwischen einem ersten und einem zweiten Ende der Vorrichtung befindet, wobei der Mittelteilabschnitt einen Bereich mit einem verminderten Außendurchmesser aufweist, der sich mindestens teilweise um den Außenumfang der Vorrichtung erstreckt und eine Arbeitsfläche zwischen der Intima des genannten Gefäßes und der aufgeblasenen Vorrichtung bildet.

31. Vorrichtung nach Anspruch 30, worin der Bereich mit dem verminderten Außendurchmesser eine Längserstreckung aufweist, die mindestens so groß ist wie seine Erstreckung um den Umfang der Vorrichtung.

32. Vorrichtung nach einem der Ansprüche 19 bis 31, worin das die Vorrichtung bildende Rohr aus mindestens zwei aufblasbaren halbrunden Wandabschnitten besteht, von denen jeder zwei größere, sich längserstreckende Ränder aufweist, die sich im wesentlichen parallel zu einem entsprechenden Rand eines benachbarten Wandabschnitts erstrecken, und wobei mindestens zwei Wandabschnitte in Fluidverbindung mit der genannten Tragstütze steht und die Tragstütze derart ausgebildet und angeordnet ist, daß sie mit den entsprechenden größeren Rändern der mindestens zwei Wandabschnitte derart ausgerichtet ist, daß die größeren Ränder von benachbarten Wandabschnitten aneinander anliegen, um dazwischen und entlang ihrer Erstreckung einen mehr oder weniger fluiddichten Verschluß zu bilden, wenn sich die Vorrichtung in einem aufgeblasenen Zustand befindet.

33. Vorrichtung nach einem der Ansprüche 1 bis 32, worin die Vorrichtung aus einem oder mehreren physiologisch annehmbaren Materialien hergestellt ist, die aus Gummi, synthetischen Gummi, Kunststoffmaterialien, Polyurethan, Polyethylen, Pluronic (RTM) und oder Blockund/oder Copolymeren dieser Stoffe ausgewählt sind.

34. Satz aus Teilen, der eine aufblasbare Vorrichtung gemäß einem der Ansprüche 1 bis 33 zum Einsetzen in ein Blut oder ein anderes Fluid führendes Gefäß sowie eine Einsetzeinrichtung für den erleichterten Zugang zur Innenseite des genannten Gefäßes umfasst.

35. Satz nach Anspruch 34, der eine Entfaltungseinrichtung zum Entfalten der Vorrichtung in ihren aufgeblasenen Zustand umfasst.

## Revendications

1. Dispositif gonflable pour être inséré, dans un état sensiblement dégonflé, dans un vaisseau sanguin ou tout autre vaisseau d'humain ou d'animal transportant un fluide biologique, le dispositif ayant un état gonflé dans lequel il définit un moyen de tube allongé à extrémité ouverte possédant une extrémité d'entrée et une extrémité de sortie, ledit tube possédant une première paroi extérieure pliable formée et agencée afin de permettre, dans ledit état gonflé, un ajustement autour de la totalité de l'intima du vaisseau utilisé dans le dispositif, et une seconde paroi intérieure pliable étroitement espacée de ladite première paroi extérieure dans ledit état gonflé, ladite seconde paroi intérieure définissant un moyen de passage sensiblement ininterrompu au sein du dispositif, par lequel du sang ou tout autre fluide biologique peut s'écouler, lors de l'utilisation du dispositif, ladite première paroi extérieure et ladite seconde paroi intérieure définissant un volume gonflable, de sorte que, lors de l'utilisation du dispositif dans un état gonflé, du sang ou tout autre fluide biologique peut passer dans le dispositif sans fuir ou couler autour de l'extérieur du dispositif, et sans que l'écoulement de celui-ci à l'intérieur du dispositif ne soit sensiblement interrompu.

2. Dispositif selon la revendication 1, dans lequel le moyen de tube allongé défini par le dispositif gonflé comprend au moins un passage, défini entre ladite extrémité d'entrée et ladite extrémité de sortie, sur la longueur longitudinale du dispositif.

3. Dispositif selon la revendication 1 ou la revendication 2, dans lequel le diamètre de ladite première paroi intérieure est supérieur à 50% du diamètre de la seconde paroi extérieure du dispositif gonflé.

4. Dispositif selon les revendications 1 ou 2, dans lequel le diamètre de ladite première paroi intérieure est de l'ordre de 90 à 95% du diamètre de la seconde paroi extérieure du dispositif gonflé.

5. Dispositif selon l'une quelconque des revendications 1 à 4, dans lequel le volume gonflable est en communication de fluide avec un canal de gonflement disposé vers l'intérieur de la paroi intérieure.

6. Dispositif selon l'une quelconque des revendications 1 à 5, dans lequel le dispositif possède une section transversale qui est généralement circulaire ou elliptique lorsqu'elle est gonflée.

7. Dispositif selon l'une quelconque des revendications 1 à 6, dans lequel le dispositif possède une section transversale irrégulière lorsqu'il est gonflé, selon la forme ou la configuration particulière du vaisseau sanguin ou du vaisseau transportant un fluide biologique endommagé dans lequel le dispositif doit être utilisé.

8. Dispositif selon l'une quelconque des revendications 1 à 7, dans lequel, lors de l'utilisation du dispositif dans un vaisseau possédant une zone lésée, le dispositif possède une longueur qui s'étend sur au moins la même longueur que ladite zone lésée dans ledit vaisseau.

9. Dispositif selon l'une quelconque des revendications 1 à 8, dans lequel le dispositif possède un canal ou un conduit de travail s'étendant au moins en partie au sein du dispositif dans lequel le canal ou le conduit de travail est formé et agencé pour recevoir à l'intérieur au moins l'un du groupe composé : d'un fluide, d'un dispositif et d'un fil de guidage.

10. Dispositif selon la revendication 9, dans lequel le canal ou le conduit de travail est l'intérieur creux d'un conduit allongé positionné de manière centrale dans le moyen de passage.

11. Dispositif selon la revendication 10, dans lequel le conduit allongé est intégralement formé avec le dispositif gonflable.

12. Dispositif selon les revendications 10 ou 11, dans lequel le conduit allongé est fixé au dispositif à au moins un point le long de celui-ci, avec les portions d'extrémité opposées du conduit allongé sortant des extrémités opposées respectives du moyen de tube défini par le dispositif gonflé.

13. Dispositif selon la revendication 12, dans lequel une première desdites portions d'extrémités sortantes est relativement longue, tandis que la seconde portion d'extrémité sort uniquement sur une très courte longueur du moyen de tube formant le dispositif gonflé.

14. Dispositif selon la revendication 13, dans lequel ladite première portion d'extrémité sortante est munie d'un premier accès d'entrée auto obturant afin de permettre l'admission de fluides ou de dispositifs, dans le canal de travail, afin de les délivrer dans le flux sanguin d'un patient.

15. Dispositif selon l'une quelconque des revendications 1 à 14, dans lequel le dispositif est formé et agencé pour coulisser sur un fil de guidage afin de faciliter l'insertion et le positionnement du dispositif.

16. Dispositif selon l'une quelconque des revendications 10 à 15, dans lequel une paroi formant le conduit allongé contient, dans l'épaisseur de celle-ci, un canal de gonflement en communication de fluide avec le volume gonflable du dispositif, et dans lequel un fluide biologiquement compatible est injecté dans le dispositif afin de gonfler le dispositif.

17. Dispositif selon la revendication 16, dans lequel une extrémité d'entrée du canal de gonflement se termine dans et est reliée à un second accès d'entrée auto obturant et prévu dans la même portion d'extrémité du conduit allongé que le premier port d'entrée.

18. Dispositif selon les revendications 16 ou 17, dans lequel une ou plusieurs parois formant ledit conduit central allongé contiennent un ou plusieurs autres canaux en communication de fluide avec d'autres accès d'entrée séparés prévus sur la même extrémité sortante du conduit allongé central, pour transporter séparément un ou plusieurs d'un fluide biologiquement compatible, de fluides d'imagerie par contraste et d'autres fluides ou dispositifs le long de celui-ci, vers, à partir ou au travers du dispositif.

19. Dispositif selon l'une quelconque des revendications 1 à 18, dans lequel le dispositif possède au moins un bras de support gonflable à l'intérieur du moyen de passage, ledit bras de support gonflable étant en communication de fluide avec ledit volume gonflable.

20. Dispositif selon la revendication 19, dans lequel le bras au moins s'étend sur le diamètre intérieur du dispositif.

21. Dispositif selon la revendication 19 ou 20, dans lequel deux, trois, quatre bras de support ou plus sont prévus.

22. Dispositif selon la revendication 19 ou 20 lorsqu'elle dépend de l'une quelconque des revendications 10 à 18, dans lequel le au moins un bras de support est formé et agencé de telle sorte que, dans l'état gonflé du dispositif, le bras s'étend radialement vers l'extérieur du conduit allongé central jusqu'à la portion extérieure annulaire du dispositif gonflé, définie entre ladite première paroi intérieure et ladite seconde paroi extérieure.

23. Dispositif selon l'une quelconque des revendications 19 à 22 lorsqu'elles dépendent de l'une quelconque des revendications 16 à 18, dans lequel le au moins un bras de support est en communication de fluide avec le canal intérieur formé dans la paroi du conduit allongé central.

24. Dispositif selon l'une quelconque des revendications 1 à 23, dans lequel le dispositif gonflé est de diamètre extérieur non uniforme le long de la dimension longitudinale de celui-ci.

25. Dispositif selon l'une quelconque des revendications 1 à 24, dans lequel le dispositif gonflé présente un profil latéral généralement en forme de sablier, possédant une portion d'étranglement relativement étroite et des portions d'extrémité relativement larges.

26. Dispositif selon la revendication 25, dans lequel le dispositif, dans son état gonflé, fournit un ajustement étanche avec l'intima du vaisseau sanguin/du vaisseau transportant un fluide biologique et dans lequel la portion d'étranglement définit un espace de travail entre la surface extérieure du dispositif et l'intima du vaisseau sanguin/du vaisseau transportant un fluide biologique.

27. Dispositif selon l'une quelconque des revendications 25 et 26, dans lequel la portion d'étranglement est gonflable séparément du reste du volume gonflable du dispositif.

28. Dispositif selon l'une quelconque des revendications 1 à 27, dans lequel le dispositif est muni d'une ou plusieurs ouvertures au sein des parois latérales dudit tube et qui sont formées et agencées pour permettre une perfusion de sang à l'intérieur, depuis le moyen de passage, dans une direction d'écoulement généralement perpendiculaire à l'axe du dispositif lorsque lesdites ouvertures sont situées à des points de ramification au sein d'un vaisseau ramifié.

29. Dispositif selon la revendication 28, dans lequel le dispositif possède un diamètre non uniforme le long de sa longueur, possédant une première portion d'extrémité relativement étroite munie desdites ouvertures, et une seconde portion relativement large.

30. Dispositif selon les revendications 24 à 29, dans lequel le dispositif possède une portion d'étranglement disposée entre les première et seconde extrémités du dispositif, dans lequel la portion d'étranglement possède une portion de diamètre extérieur réduit s'étendant au moins partiellement autour de la circonférence extérieure du dispositif et définit une zone de travail entre l'intima dudit vaisseau et le dispositif gonflé.

31. Dispositif selon la revendication 30, dans lequel la portion de diamètre extérieur réduit possède une étendue longitudinale au moins aussi longue que son étendue autour de la circonférence du dispositif.

32. Dispositif selon l'une quelconque des revendications 19 à 31, dans lequel le tube formant le dispositif est formé d'au moins deux portions de paroi gonflables semi-circulaires, chaque portion de paroi possédant deux bords principaux s'étendant longitudinalement qui s'étendent sensiblement parallèlement à un bord correspondant d'une portion de paroi adjacente ; lesdites au moins deux portions de paroi étant en communication de fluide avec ledit bras de support, dans lequel ledit bras de support est formé et agencé afin d'aligner les bords principaux respectifs des au moins deux portions de paroi de telle sorte que les bords principaux des portions de paroi adjacentes aboutent l'un contre l'autre afin de former un joint plus ou moins étanche au fluide entre ceux-ci et le long de l'étendue de ceux-ci lorsque le dispositif est dans un état gonflé.

33. Dispositif selon l'une quelconque des revendications 1 à 32, dans lequel le dispositif est fabriqué à partir d'un ou plusieurs matériaux physiologiquement acceptables choisis parmi le groupe comprenant le caoutchouc, le caoutchouc synthétique, les matériaux plastiques, le polyuréthane, le polyéthylène, le Pluronic (marque déposée), et/ou des polymères séquencés, et/ou des copolymères de ceux-ci.

34. Ensemble de pièces comprenant un dispositif gonflable selon l'une quelconque des revendications 1 à 33 pour l'insertion dans un vaisseau sanguin ou tout autre vaisseau transportant un fluide, et un moyen d'insertion pour faciliter l'accès à l'intérieur dudit vaisseau.

35. Ensemble de pièces selon la revendication 34, qui comprend un moyen de déploiement pour déployer le dispositif dans son état gonflé.
